# EUROPEAN PATENT SPECIFICATION

(11) **EP 4 342 409 B1**
(45) Date of publication and mention of the grant of the patent: **06.05.2026**
(21) Application number: 21940722.8
(22) Date of filing: 18.05.2021
(51) Int. Cl.: A61B 34/10, A61B 34/20, A61B 34/00, A61B 34/37, A61B 90/00

(54) **FORCE SENSE DISPLAY DEVICE AND PROGRAM**
KRAFTERFASSUNGSANZEIGEVORRICHTUNG UND PROGRAMM
DISPOSITIF D'AFFICHAGE DE DÉTECTION DE FORCE ET PROGRAMME

(43) Date of publication of application: 27.03.2024
(73) Proprietor: RIVERFIELD Inc., Tokyo 107-0052 (JP)
(72) Inventor: NISHIHARA, Teruyuki, Tokyo 107-0052 (JP)
(74) Representative: Adares PartGmbB
(86) International application number: PCT/JP2021/018815
(87) International publication number: WO 2022/244105

(56) References cited:
- WO-A1-2020/176128
- WO-A1-2020/189110
- WO-A1-2020/189120
- JP-A- 2001 104 333
- JP-A- 2020 146 374
- JP-A- H04 261 787
- US-A1- 2020 188 045

## Description

### TECHNICAL FIELD

The present disclosure relates to a force sense display device, an unclaimed force sense display method, and a program.

### BACKGROUND ART

In endoscopic surgery, laparoscopic surgery, and the like, use of surgical tools such as an endoscope, a laparoscope, and forceps, is becoming widespread. Hereinafter, a surgical tool such as an endoscope, a laparoscope, or forceps is also referred to as a "surgical instrument". Endoscopic surgery, laparoscopic surgery, and the like, using the surgical instrument, are also referred to as "endoscopic surgery and the like".

In endoscopic surgery and the like, in addition to surgeries using a surgical instrument held in the hand of an operator such as a doctor, there are also surgeries performed using a medical robot performing surgical assistance as disclosed in, for example, Patent Document 1. The surgery using a medical robot that performs surgical assistance is also referred to as a robotic-assisted surgery.

### PRIOR ART DOCUMENTS

### PATENT DOCUMENTS

Patent Document 1: Japanese Unexamined Patent Application Publication No. 2001-104333
Patent Document 2: US 2020/188045 A1
Patent Documents 3: WO 2020/189110 A1
Patent Document 4: WO 2020/176128 A1
Patent Document 5: WO 2020/189120 A1

### SUMMARY OF THE INVENTION

### PROBLEMS TO BE SOLVED BY THE INVENTION

In endoscopic surgery and the like, it is known that operators find it difficult to know a force exerted by a surgical instrument on tissues such as organs. For example, it is known that operators find it difficult to know forces such as a force with which a surgical instrument presses tissue, and a force with which a surgical instrument holds tissue. As a result, more force than intended is applied to the tissue, which may lead to damage to the tissue. Hereinafter, information relating to a force exerted by a surgical instrument on tissues such as organs is referred to as "force amount information".

As a method for an operator to know the force amount information described above, it is known to use force amount display equipment specialized for displaying the force amount information to the operator, separately from image display equipment for displaying images captured by an endoscope. This use method requires the operator to move a line of sight from the image display equipment to the force amount display equipment, in order for the operator to know the force amount information. Thus, there is a concern that tissue may be accidentally damaged during movement of the line of sight.

Further, a method is known of displaying, on an image display unit, force amount information superimposed. In this method, the force amount information is superimposed on a portion where tissue is displayed on the image display unit, thereby to hide the tissue, thus making it difficult to visually recognize the hidden tissue. Thus, there is a concern that the tissue, which is difficult to be visually recognized, may be damaged accidentally.

Patent Document 1 described above discloses a technique of displaying force amount information superimposed on a part of an instrument corresponding to the surgical instrument displayed on an image display unit. Specifically, Patent Document 1 discloses a technique of superimposing and displaying an indicator representing a magnitude of a force to hold tissue, and information on a joint angle of the instrument and on an angle of rotation thereof. There is a concern that the information to be displayed in this case is insufficient and thus it is difficult to predict damage to the tissue.

Patent Document 2 describes a robotic surgical system comprising a visual display including a force feedback meter, and computer system which can position a surgical tool, an end effector relative to a surgical site, and an image capture device, the images thereof being displayed by the visual display.

Patent Document 3 describes a medical robot system with a control device which can create image information about an external force as an image in association with the location where the external force was measured, and a display device which displays the image information.

Patent Document 4 describes methods for measuring a force by a tracking system, wherein numerical values for the magnitude and/or direction may be displayed to a user, and if the user is wearing an augmented reality headset, feedback information pertaining to the magnitude may include a visual indication overlaid over the tool.

Patent Document 5 describes a force sense display device having an input unit which is connected to a shaft sensor that measures an external force applied to a shaft, a joint sensor that measures the external force applied to the joint portion, a surgical tool sensor that measures the opening and closing force applied to the outside by a surgical tool and an image generating unit which generates image information based on video and image signals input from an endoscope unit and signals input from the shaft sensor, the joint sensor, and the surgical tool sensor.

The present disclosure discloses examples of a force sense display device, a force sense display method, and a program that can easily reduce damage to tissue.

### MEANS FOR SOLVING THE PROBLEMS

The problem is solved by a force sense display device according to claim 1 and a program according to claim 6.

One aspect of the present disclosure provides a force sense display device comprising an image obtainer configured to obtain captured-image information that is output from an image-capturing section configured to capture an image of an affected part; a force sense obtainer configured to obtain force sense information comprising information on a magnitude of and a direction of a force acting on at least one surgical instrument for treatment of the affected part; a generator configured to generate a force sense representation based on the force sense information, the force sense representation being a mode of representation to be displayed on a display section and comprising a force amount representation that represents the magnitude of the force and a direction representation that represents the direction of the force; a detector configured to detect a surgical instrument area where the surgical instrument is displayed in an image displayed on the display section, based on the captured-image information; and a superimposing section configured to generate display information for display, on the display section, of the surgical instrument area with the force sense representation superimposed thereon.

One aspect of the present disclosure provides an unclaimed force sense display method in a force sense display device including an image obtainer, a force sense obtainer, a generator, a detector, and a superimposing section, the display method comprising: an image obtaining step of obtaining captured-image information by the image obtainer, the captured-image information being output from an image-capturing section capturing an image of an affected part; a force sense obtaining step of obtaining force sense information by the force sense obtainer, the force sense information comprising information on a magnitude of and a direction of a force acting on at least one surgical instrument for treatment of the affected part; a generating step of generating a force sense representation by the generator based on the force sense information, the force sense representation being a mode of representation to be displayed on a display section and comprising a force amount representation that represents the magnitude of the force and a direction representation that represents the direction of the force; a detecting step of detecting, by the detector, a surgical instrument area where the surgical instrument is displayed in an image displayed on the display section, based on the captured-image information; and a superimposing step of generating, by the superimposing section, display information for display, on the display section, of the surgical instrument area with the force sense representation superimposed thereon.

One aspect of the present disclosure provides a program for causing a computer to execute an image obtaining function of obtaining captured-image information that is output from an image-capturing section capturing an image of an affected part; a force sense obtaining function of obtaining force sense information comprising information on a magnitude of and a direction of a force acting on at least one surgical instrument for treatment of the affected part; a generating function of generating a force sense representation based on the force sense information, the force sense representation being a mode of representation to be displayed on a display section and comprising a force amount representation that represents the magnitude of the force and a direction representation that represents the direction of the force; a detecting function of detecting a surgical instrument area where the surgical instrument is displayed in an image displayed on the display section, based on the captured-image information; and a superimposing function of generating display information for display, on the display section, of the surgical instrument area with the force sense representation superimposed thereon.

This program may be stored in a ROM, a RAM, or the like incorporated in a computer, or may be loaded into the computer from the ROM, the RAM, or the like, or may be loaded into the computer via a network. The above-described program may be recorded in any form of computer-readable recording medium. Examples of the recording medium include a portable semiconductor memory (for example, a USB memory and the like).

According to the force sense display device, the force sense display method, and the program configured as such, the force sense representation containing the force amount representation that represents the magnitude of the force and the direction representation that represents the direction of the force is displayed in a manner superimposed on the surgical instrument area where the surgical instrument is displayed in the image displayed on the display section. Accordingly, it is easier to know the magnitude and direction of the force applied by the surgical instrument to the affected part. In other words, it is easier to know damage caused by the surgical instrument to tissue in the affected part.

Further, the force sense representation is displayed in a manner superimposed on the surgical instrument area, and the affected part displayed on the display section is less likely to be hidden by the force sense representation. Accordingly, the surgical instrument and the affected part are less likely to accidentally touch each other, and the damage to the tissue due to the touch is less likely to be reduced.

In one aspect of the present disclosure, preferably, the generator generates the force sense representation comprising the force amount representation that represents the magnitude of the force using at least one of a color type, a color brightness/darkness, or a transparency.

The force sense display device configured as such represents the magnitude of the force using one of a color type, a color brightness/darkness, or a transparency, or a combination thereof, thereby making it easier to intuitively know the magnitude of the force.

In one aspect of the present disclosure, preferably, the generator generates the force sense representation comprising the force amount representation that represents the magnitude of the force using a numerical value.

The force sense display device configured as such is capable of accurately representing the magnitude of the force by representing the magnitude of the force using numerical values.

In one aspect of the present disclosure, preferably, the generator generates the force sense representation comprising the force amount representation that represents the magnitude of the force using a size of the numerical value to be displayed.

The force sense display device configured as such represents, using the size of the numerical values, the magnitude of the force, which enables the magnitude of the force to be accurately represented and more easily known intuitively, compared to a case in which the magnitude of the force is represented using numerical values alone.

In one aspect of the present disclosure, it is preferable to further comprise a storage section configured to store at least one of a color tone or a pattern of the surgical instrument. Preferably, the detector detects the surgical instrument area in the image displayed on the display section, based on the at least one of the color tone or the pattern of the surgical instrument stored in the storage section.

The force sense display device configured as such detects the surgical instrument area based on the at least one of the color tone or the pattern of the surgical instrument, so that it is easier to detect the surgical instrument area. Further, it is easier to more accurately detect the surgical instrument area.

In one aspect of the present disclosure, the force sense information comprises information on a magnitude of and a direction of a first force acting on a shaft of the surgical instrument extending in a rod shape, the generator generates a first force sense representation comprising a first force amount representation that represents the magnitude of the first force and a first direction representation that represents the direction of the first force, the detector detects, as a first surgical instrument area, an area where the shaft is displayed in the image displayed on the display section, and the superimposing section generates display information for display, on the display section, of the first surgical instrument area with the first force sense representation superimposed thereon.

According to the force sense display device configured as such, the first force sense representation relating to the shaft of the surgical instrument is displayed in a manner superimposed on the first surgical instrument area, so that it is easier to know the magnitude of and the direction of the force applied by the shaft to the affected part.

In one aspect of the present disclosure, the force sense information comprises information on a magnitude of and a direction of a second force applied to an end effector that is provided on an end of the shaft and used for treatment of the affected part, the generator generates a second force sense representation comprising a second force amount representation that represents the magnitude of the second force and a second direction representation that represents the direction of the second force, the detector detects, as a second surgical instrument area, an area where the end effector is displayed in the image displayed on the display section, and the superimposing section generates display information for display, on the display section, of the second surgical instrument area with the second force sense representation superimposed thereon.

According to the force sense display device configured as such, the second force sense representation relating to the end effector of the surgical instrument is displayed in a manner superimposed on the second force sense representation where the end effector is displayed, so that it is easier to know the magnitude of and the direction of the force applied by the end effector to the affected part.

### EFFECTS OF THE INVENTION

The force sense display device, the force sense display method, and the program of the present disclosure produce effects that it is easier to know the magnitude of and the direction of the force applied by the surgical instrument to the affected part, and it is easier to reduce damage to tissue.

### BRIEF DESCRIPTION OF THE DRAWINGS

FIG. 1 is a block diagram explaining a configuration of a medical robot according to a first embodiment of the present disclosure.
FIG. 2 is a diagram explaining a configuration of an instrument displayed on an endoscope monitor.
FIG. 3 is a block diagram explaining a configuration of a force sense display device.
FIG. 4 is a diagram explaining a first force sense representation and a second force sense representation displayed on the endoscope monitor.
FIG. 5 is a diagram explaining another display example of a first direction representation.
FIG. 6 is a diagram explaining still another display example of the first direction representation.
FIG. 7 is a diagram explaining still another display example of the first direction representation.
FIG. 8 is a diagram explaining still another display example of the first direction representation.
FIG. 9 is a flowchart explaining arithmetic processes to perform a force sense representation.
FIG. 10 is a diagram explaining a configuration of the force sense display device when the force sense display device is used in laparoscopic surgery.
FIG. 11 is a block diagram explaining a configuration of a force sense display device according to a modified example of the present disclosure.

### EXPLANATION OF REFERENCE NUMERALS

12...endoscope monitor (display section)
21R, 21L, 121R, 121L...instruments (surgical instruments)
22R, 22L...shafts
23R, 23L...end effectors
35... image-capturing section
50, 50A...force sense display devices
51...image obtainer
52...force sense obtainer
53...generator
54, 54A...detectors
55...superimposing section
57A...storage section
41R, 41L... first force amount representations (force amount representations)
42R, 42L...first direction representations (direction representations)
46R, 46L...second force amount representations (force amount representations)
27R, 27L...first surgical instrument areas (surgical instrument areas)
28R, 28L...second surgical instrument areas (surgical instrument areas)
S101...image obtaining step
S102...detecting step
S103...force sense obtaining step
S104... generating step
S105...superimposing step

### MODE FOR CARRYING OUT THE INVENTION

Embodiments described below represent examples of embodiments that fall within the technical scope of the present disclosure. The technical scope of the present disclosure is not limited to the specific configurations, structures, and others shown in the embodiments described below. The invention is defined by the claims and in particular by independent claims 1 and 6.

Arrows, oblique lines, and others indicating directions in each figure are described to facilitate knowing of the relationship between the figures, and the shape of each member or portion. Thus, the technical scope of the present disclosure is not limited to the directions shown in each figure. The figures with the oblique lines do not necessarily show sectional views.

At least one member or one portion is provided for a member or a portion at least described with a reference numeral, except when the member or the portion is explicitly described as "one member" or the like. In other words, if there is no mention of "one member" or the like, two or more of such members may be provided. A force sense display device and a medical robot of the present disclosure comprise components, such as members and portions at least described with reference numerals, and illustrated structural portions.

### [First Embodiment]

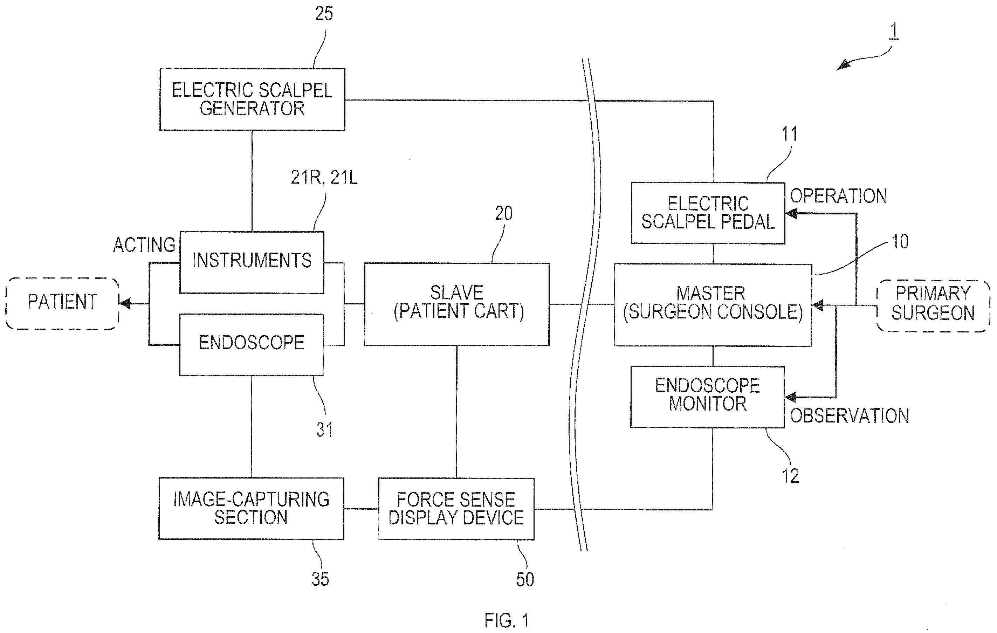

A force sense display device 50 and a medical robot 1 including the force sense display device 50 according to a first embodiment of the present disclosure will be described with reference to FIG. 1 to FIG. 9. The medical robot 1 of the present embodiment is a robot that performs surgical assistance in endoscopic surgery and the like.

The present embodiment describes an example case where the force sense display device 50 is integrally configured with the medical robot 1. The force sense display device 50 may be configured to include a separated body detachably attachable to the medical robot 1.

As shown in FIG. 1, the medical robot 1 is provided with a master 10, a slave 20, and the force sense display device 50.

The master 10 is a surgeon console by which a primary surgeon performing surgery using the medical robot 1 operates the slave 20. The master 10 is connected to the slave 20 to be able to communicate signals relating to the operation therewith.

The present embodiment describes an example case where the master 10 is placed in a room different from the room where the slave 20 is placed. The master 10 may be placed in the same room as the room where the slave 20 is placed.

The master 10 is provided with at least an electric scalpel pedal 11 and an endoscope monitor 12. The master 10 is also provided with an operating section (not shown) operated by the primary surgeon, and used for operation, for example, moving or grasping two instruments 21R, 21L, which will be described below.

The electric scalpel pedal 11 is a device operated by the primary surgeon and controlling movement of an electric scalpel generator 25, which will be described below. The present embodiment describes an example case where the electric scalpel pedal 11 is a device disposed at the primary surgeon's feet and stepped on by the primary surgeon to control the movement of the electric scalpel generator 25. The electric scalpel pedal 11 may include a configuration other than the configuration stepped on by the primary surgeon.

The endoscope monitor 12 is a display section used when the primary surgeon performs surgery and displaying an image captured by an endoscope 31, which will be described below. As the endoscope monitor 12, commonly-used display equipment may be used, and its display format is not particularly limited. The endoscope monitor 12 may be attached to the master 10 and configured integrally, or may be attached to a device separate from the master 10.

The slave 20 is a patient cart that is placed in a room where the surgery is performed and that performs surgery in accordance with an operation which is input by the primary surgeon through the master 10. The slave 20 is provided with a mechanism to which the instruments 21R, 21L (described below) and the endoscope 31 are attached, a mechanism for moving the instruments 21R, 21L and the endoscope 31, a mechanism for driving them, and the like.

The present embodiment describes an example configuration in which the mechanisms using air pressures for moving and driving the instruments 21R, 21L and the endoscope 31 are provided to the slave 20. The slave 20 may be provided with any other mechanisms using an electric motor, other than the mechanisms for moving and driving the instruments 21R, 21L and the endoscope 31.

The slave 20 is provided with the two instruments 21R, 21L, and one endoscope 31. The present embodiment describes an example configuration in which the two instruments 21R, 21L and one endoscope 31 are attached to the slave 20.

The number of instruments attachable to the slave 20 may be one, three or more. The number of endoscopes attachable to the slave 20 may be two or more.

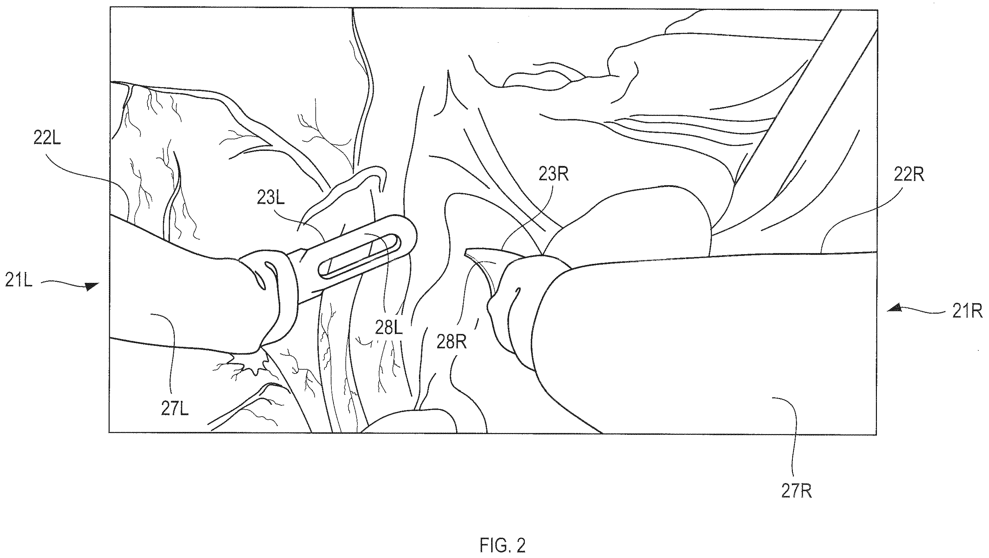

The instruments 21R, 21L are devices to be attached to the slave 20, and surgical instruments to perform various treatments in the surgery in accordance with the operation by the primary surgeon. As shown in FIG. 2, the instrument 21R includes at least a shaft 22R and an end effector 23R. The instrument 21L includes at least a shaft 22L and an end effector 23L.

The shaft 22R and the shaft 22L each are a member formed into a rod shape. The end effector 23R is disposed at an end of the shaft 22R, and 23L is disposed at an end of the shaft 22R. The present embodiment describes an example configuration in which the ends of the shafts 22R, 22L where the end effectors 23R, 23L are respectively disposed, are members to be inserted into the abdominal cavity of a patient. The shaft 22R and the shaft 22L may have the same shape in diameter and length, or at least one of the shaft 22R and the shaft 22L may have a different shape.

The present embodiment describes an example configuration in which the end effector 23R is an electric scalpel cutting an affected part, and the end effector 23L is a forceps for grasping the affected part and dissecting adhered affected-areas. Further, an example configuration is described in which the end effector 23R is connected to receive an electric power supply from the electric scalpel generator 25. The end effectors 23R, 23L may include any configuration, other than an electric scalpel and a forceps for treatment of an affected part.

As shown in FIG. 1, the electric scalpel generator 25 is a device operably connected to the electric scalpel pedal 11 and supplying electric power to the end effector 23R. The electric power supplied from the electric scalpel generator 25 is also used for hemostasis or the like of an affected part dissected by the end effector 23R. As the electric scalpel generator 25, a commonly-used device for supplying electric power to an end effector of an electric scalpel may be used.

The endoscope 31 is a device to be attached to the slave 20, inserted into a body cavity of the patient, and used for capturing an image of an affected part thereof. The endoscope 31 is provided with an image-capturing section 35 used for capturing an image.

The image-capturing section 35 is provided with elements such as an image-capturing element that converts the image of the affected part introduced by the endoscope 31 to an electrical signal and outputs the converted image as captured-image information, a light-emitting element that emits light for irradiating the affected part, and a computing element used for controlling the image-capturing element and the light-emitting element.

As the image-capturing element, a commonly-used image-capturing element such as a solid image-capturing element (e.g., CCD: Charge Coupled Device) may be used. As the light-emitting element, a commonly-used light-emitting element such as an LED (Light Emitting Diode) may be used.

The present embodiment describes an example configuration in which the image-capturing element and the light-emitting element are provided at the image-capturing section 35 different from the endoscope 31. However, at least one of the image-capturing element or the light-emitting element may be provided at the endoscope 31.

As shown in FIG. 1, the force sense display device 50 is a device arranged between the slave 20 and the endoscope monitor 12 and between the image-capturing section 35 and the endoscope monitor 12. Further, the force sense display device 50 is a device that generates display information in which a force sense representation representing a magnitude of and a direction of a force acting on each of the instruments 21R, 21L is superimposed on the captured-image information that is output from the image-capturing section 35.

The force sense display device 50 is an information processing device such as a processor including a CPU (Central Processing Unit), a ROM, a RAM, an input/output interface, etc., a microcomputer, a personal computer, or a server.

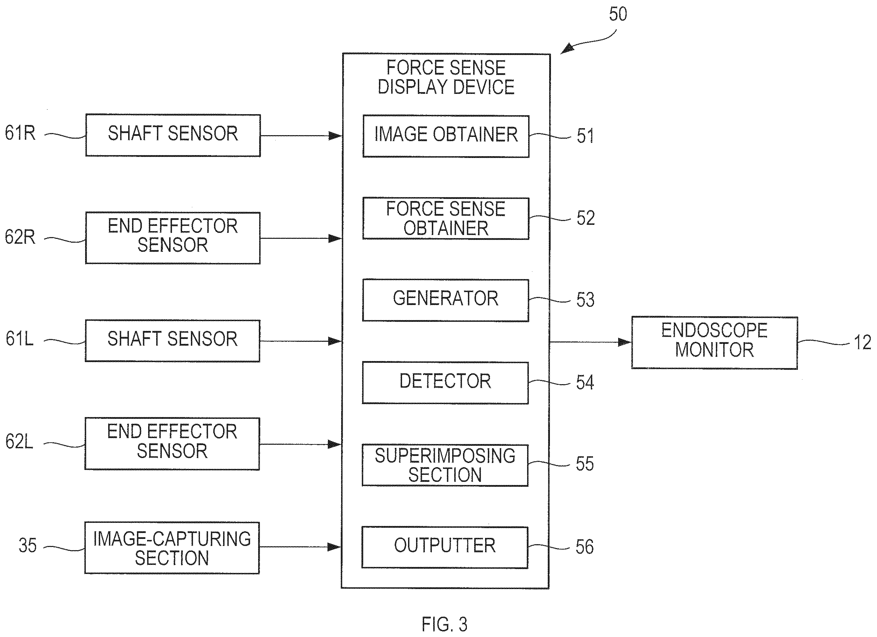

A program stored in a storage device such as the ROM causes the CPU, the ROM, the RAM, and the input/output interface to cooperate with each other and function as at least an image obtainer 51, a force sense obtainer 52, a generator 53, a detector 54, a superimposing section 55, and an outputter 56, as shown in FIG. 3.

The image obtainer 51 is an interface that obtains the captured-image information that is output from the image-capturing section 35. The image obtainer 51 is connected to the image-capturing section 35 in an information-communicable manner. Further, the image obtainer 51 is connected to the detector 54 and the superimposing section 55 to allow output of the obtained captured-image information.

The force sense obtainer 52 is an interface that obtains force sense information containing information on the magnitude of and the direction of the force acting on each of the instruments 21R, 21L. The force sense obtainer 52 is connected to a shaft sensor 61R, an end effector sensor 62R, a shaft sensor 61L, and an end effector sensor 62L in an information-communicable manner. The force sense obtainer 52 is connected to the generator 53 to allow output of the obtained force sense information. Hereinafter, the force acting on the shaft of the instrument is referred to as a first force, and the force acting on the end effector is referred to as a second force.

The shaft sensor 61R is a sensor that measures force sense information containing information on a magnitude of and a direction of the first force acting on the shaft 22R of the instrument 21R. The end effector sensor 62R is a sensor that measures force sense information containing information on a magnitude of the second force acting on the end effector 23R of the instrument 21R.

The shaft sensor 61L is a sensor that measures force sense information containing information on a magnitude of and a direction of the first force acting on the shaft 22L of the instrument 21L. The end effector sensor 62L is a sensor that measures force sense information containing information on a magnitude of the second force acting on the end effector 23L of the instrument 21L.

The present embodiment describes an example configuration in which the shaft sensor 61R, the end effector sensor 62R, the shaft sensor 61L, and the end effector sensor 62L are sensors that measure respective air pressures in the mechanisms, which are provided to the slave 20, for moving and driving the instruments 21R, 21L and the endoscope 31.

The shaft sensor 61R, the end effector sensor 62R, the shaft sensor 61L, and the end effector sensor 62L may be any other sensor, such as a pressure sensor, used when a magnitude of and a direction of a force is measured.

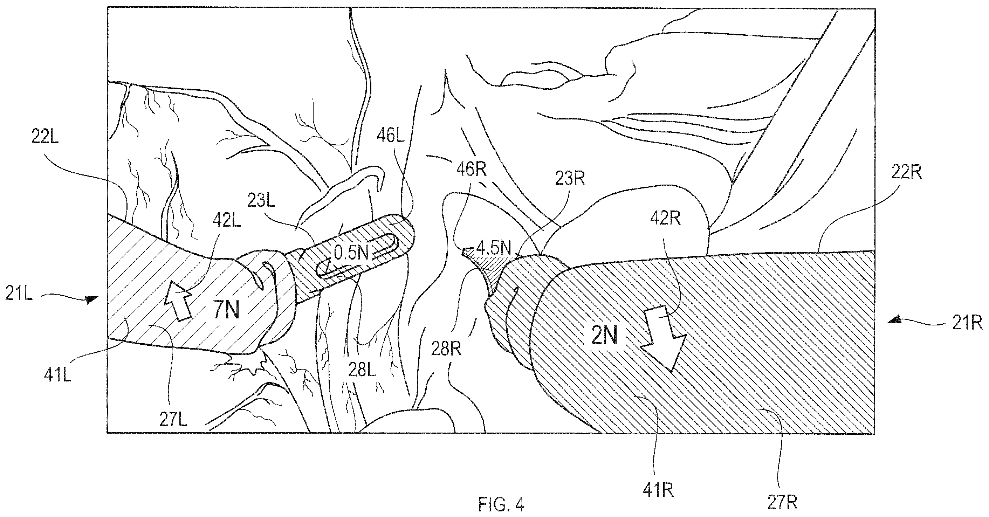

The generator 53 is a calculator that generates a force sense representation that is a force amount representation and a direction representation, based on the force sense information obtained by the force sense obtainer 52. The present embodiment describes an example case where the generator 53 generates a first force sense representation and a second force sense representation (see FIG. 4). The first force sense representation contains a first force amount representation 41R representing the magnitude of the first force and a first direction representation 42R representing the direction of the first force. The first force sense representation contains a first force amount representation 41L representing the magnitude of the first force and a first direction representation 42L representing the direction of the first force. The second force sense representation contains a second force amount representation 46R representing the magnitude of the second force. The second force sense representation contains a second force amount representation 46L representing the magnitude of the second force. The generator 53 is connected to the superimposing section 55 to allow output of the generated first force sense representation and second force sense representation.

Each of the first force amount representations 41R, 41L is a mode of representation for display on the endoscope monitor 12 and represents the magnitude of the first force. Each of the first direction representations 42R, 42L is a mode of representation for display on the endoscope monitor 12 and represents the direction of the first force.

Each of the second force representations 46R, 46L is a mode of representation for display on the endoscope monitor 12 and represents the magnitude of the second force. Details of the first force amount representations 41R, 41L, the first direction representations 42R, 42L, and the second force amount representations 46R, 46L will be described below.

The detector 54 is a calculator that detects a surgical instrument area based on the obtained captured-image information. The surgical instrument area is an area where each of the instruments 21R, 21L is displayed when the captured-image information is displayed on the endoscope monitor 12.

As shown in FIG. 2, the surgical instrument areas contain first surgical instrument areas 27R, 27L where the shafts 22R, 22L are respectively displayed, and second surgical instrument areas 28R, 28L where the end effectors 23R, 23L are respectively displayed. The detector 54 is connected to the superimposing section 55 to allow output of information relating to the detected first surgical instrument areas 27R, 27L and second surgical instrument areas 28R, 28L.

The present embodiment describes an example case where the detector 54 executes an image analysis process on the captured-image information to detect the first surgical instrument areas 27R, 27L and the second surgical instrument areas 28R, 28L. As the image analysis process, a commonly-used process method for detecting a specific area based on captured-image information may be used.

The superimposing section 55 is a calculator that generates display information in which the generated first force sense representation and second force sense representation are superimposed on the detected first surgical instrument area 27R and second surgical instrument area 28R, and first surgical instrument area 27L and second surgical instrument area 28L, respectively. The superimposing section 55 is connected to the outputter 56 to allow output of the generated display information. The display information is information on images to be displayed on the endoscope monitor 12. As the process to superimpose the force sense representation on the surgical instrument area at the superimposing section 55, a commonly-used superimposing process may be used.

As shown in FIG. 3, the outputter 56 is an interface that outputs the generated display information to the endoscope monitor 12. The outputter 56 is connected to the endoscope monitor 12 in an information-communicable manner. As the outputter 56, an interface having a general configuration may be used.

Next, the following describes an unclaimed method for displaying a force sense by the force sense display device 50 having the aforementioned configuration of the medical robot 1. Herein, to simplify the explanation, the following describes processing steps to display a force sense when the force is applied to each of the shaft 22R and end effector 23R of the instrument 21R. In a case where the force is applied to each of the shaft 22L and end effector 23L of the instrument 21L, the processing steps are similarly executed.

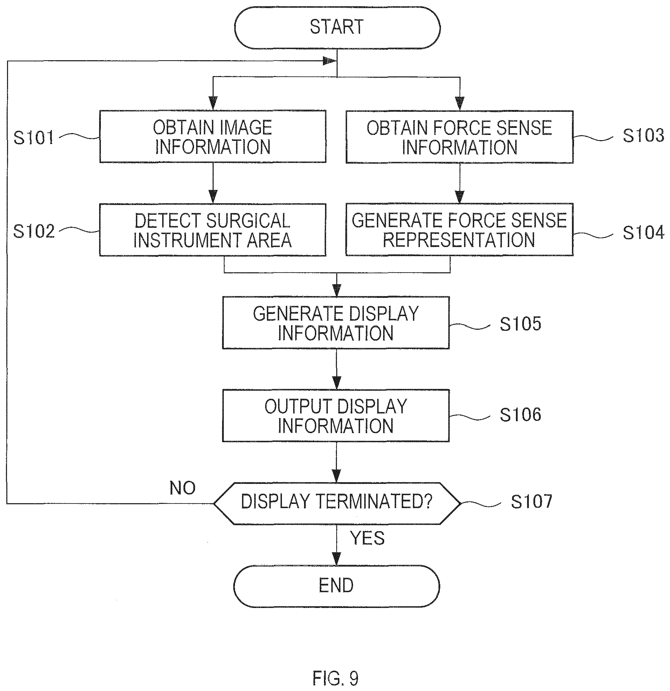

The processing steps in FIG. 9, which will be described below, are executed by the program stored in the storage device such as the ROM that causes the CPU, the ROM, the RAM, and the input/output interface in the force sense display device 50 to cooperate with each other.

As shown in FIG. 9, when the process to display the force sense is started by the force sense display device 50, the image obtainer 51 executes a process to obtain image information (S101: image obtaining step). In the present embodiment, as shown in FIG. 1, first, the image of the affected part introduced by the endoscope 31 is incident on the image-capturing section 35.

The image-capturing section 35 converts the incident image to the captured-image information that is an electrical signal, and then outputs the converted captured-image information. As shown in FIG. 3, the captured-image information that is output is obtained by the image obtainer 51. The image obtainer 51 outputs the obtained captured-image information to the detector 54 and to the superimposing section 55.

As shown in FIG. 9, when the image information is obtained, the detector 54 executes a process to detect the surgical instrument area (S102: detecting step). In the present embodiment, as shown in FIG. 2, the detector 54 executes an arithmetic process to detect the first surgical instrument area 27R and the second surgical instrument area 28R based on the obtained captured-image information.

As the arithmetic process to detect the first surgical instrument area 27R and the second surgical instrument area 28R, a commonly-used detection process for detecting a specified area based on the image information may be used. As shown in FIG. 3, the detected first surgical instrument area 27R and second surgical instrument area 28R are output from the detector 54 to the superimposing section 55.

On the other hand, as shown in FIG. 9, the force sense obtainer 52 executes an obtaining process to obtain the force sense information (S103: force sense obtaining step). In the present embodiment, as shown in FIG. 2 and FIG. 3, when the force is applied to the shaft 22R of the instrument 21R, the shaft sensor 61R measures the magnitude of and the direction of the first force acting on the shaft 22R. The shaft sensor 61R outputs the force sense information containing information on the magnitude and direction of the first force.

When the force is applied to the end effector 23R of the instrument 21R, the end effector sensor 62R measures the magnitude of the second force acting on the end effector 23R. The end effector sensor 62R outputs the force sense information containing information on the magnitude of the second force.

The force sense obtainer 52 obtains the force sense information that is output from each of the shaft sensor 61R and the end effector sensor 62R. The obtained force sense information is output from the force sense obtainer 52 to the generator 53.

As shown in FIG. 9, when the force sense information is obtained, the generator 53 executes an arithmetic process to generate the first force sense representation and the second force sense representation (S104: generating step). In the present embodiment, as shown in FIG. 4, the generator 53 executes the arithmetic process to generate the first force amount representation 41R, the first direction representation 42R, and the second force amount representation 46R based on the force sense information that is input. The generated first force amount representation 41R, first direction representation 42R, second force amount representation 46R are output from the generator 53 to the superimposing section 55, as shown in FIG. 3.

The present embodiment describes an example case where the first force amount representation 41R comprises an area representation that is at least a part of the first surgical instrument area 27R and that is color-changeable in accordance with the magnitude of the force and also comprises a representation that is a numerical value indicating the magnitude of the force and that is size-changeable in accordance with the magnitude of the force. As a more specific example, a case is described in which such a color-changeable area has the same shape as the first surgical instrument area 27R. Herein, the size of the numerical value refers to a size of a character representing the numerical value indicating the magnitude of the force.

Further, as an example, a case is described in which the second force amount representation 46R comprises an area representation that is at least a part of the second surgical instrument area 28R and that is color-changeable in accordance with the magnitude of the force and also comprises a representation that is a numerical value indicating the magnitude of the force and that is size-changeable in accordance with the magnitude of the force. As a more specific example, a case is described in which such a color-changeable area has the same shape as the second surgical instrument area 28R.

The present embodiment describes an example case where a method of changing a color in accordance with the magnitude of the force is a method of changing the color from green to yellow, and further changing from yellow to red, as the magnitude of the force increases. The order of changing colors and the selection of colors to be displayed are not limited to the examples of the present embodiment.

As an example, a case is described in which a method of changing the size of the numerical value in accordance with the magnitude of the force is a method of displaying the numerical value in a larger size in direct proportion to the numerical value to be displayed. The method of changing the size is not limited to the examples of the present embodiment.

In the first force amount representation 41R and the second force amount representation 46R, each of the area representation that is at least a part of the first surgical instrument area 27R and that is color-changeable in accordance with the magnitude of the corresponding force and the area representation that is at least a part of the second surgical instrument area 28R and that is color-changeable in accordance with the magnitude of the corresponding force may be an area representation where a color brightness/darkness varies in accordance with the magnitude of the force. For example, the representation may vary from a dark color to a bright color in accordance with the magnitude of the force.

In the first force amount representation 41R and the second force amount representation 46R, each of the area representation that is at least a part of the first surgical instrument area 27R and that is color-changeable in accordance with the magnitude of the corresponding force and the area representation that is at least a part of the second surgical instrument area 28R and that is color-changeable in accordance with the magnitude of the corresponding force may be an area representation whose transparency varies in accordance with the magnitude of the force. For example, the representation may vary from transparent to translucent through opaque in accordance with the magnitude of the force.

The present embodiment describes an example case where the first direction representation 42R is an arrow indicating the direction of the force. The first direction representation 42R may be any commonly-used representation for indicating a direction, other than the arrow.

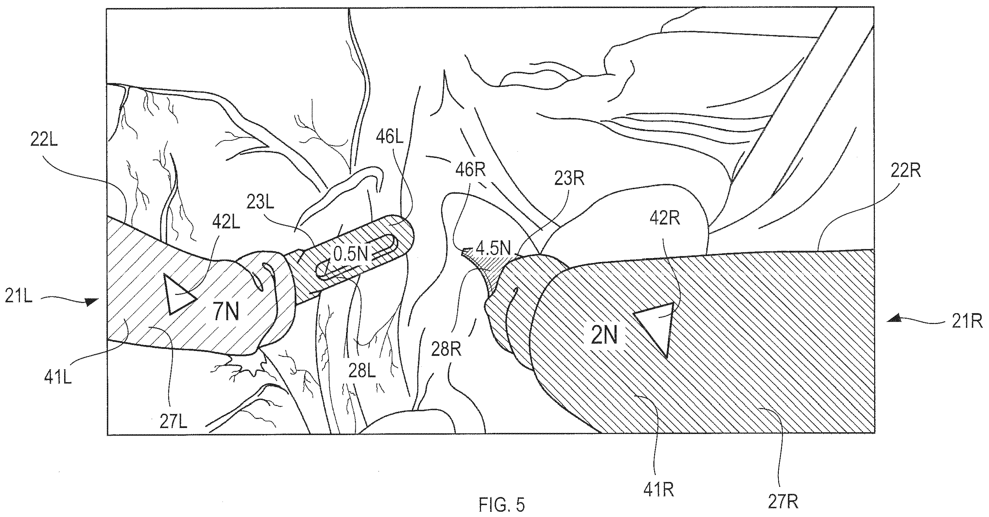

For example, as shown in FIG. 5, each of the first direction representations 42R, 42L may be shaped as a triangle indicating the direction of the corresponding force. The triangle is an isosceles triangle with two equal sides longer than one base. A direction with an apex angle between the two equal sides represents the direction of the force.

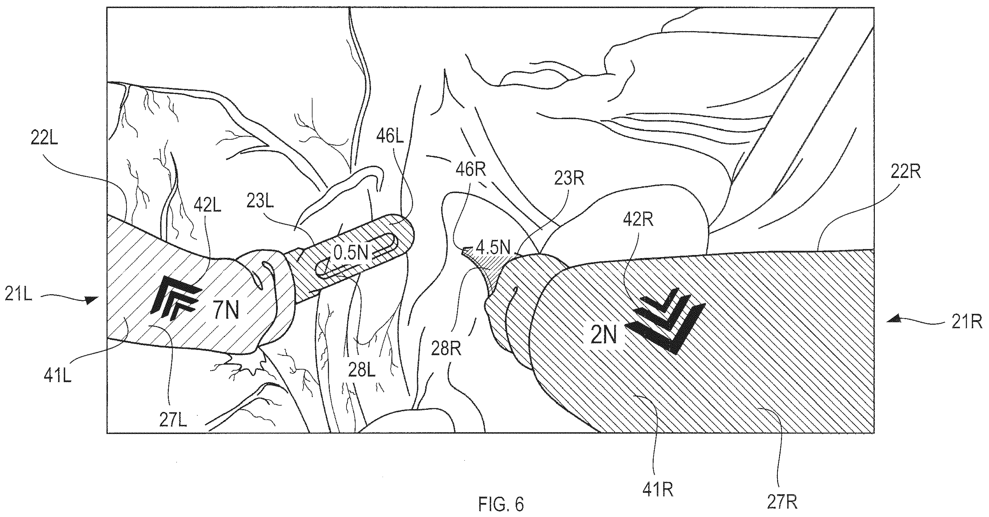

As shown in FIG. 6, each of the first direction representations 42R, 42L may be represented as a group of three L-shapes indicating the direction of the corresponding force, in other words, a group of a small L-shape, a medium L-shape, and a large L-shape. The number of L-shapes may be one, two, four or more.

In the group, the L-shapes with different sizes are aligned in order of the size. The direction indicated by a corner of each L-shape represents the direction of the force. If there are two or more L-shapes, the direction of the force of each L-shape is also represented by a direction of arrangement from a small L-shape to a large L-shape.

A representation state of the group of the three L-shapes may remain constant regardless of a passage of time, or may be changed over time. Specifically, as time passes, the representation may change from displaying only the small L-shape, through displaying the small and medium L-shapes, to displaying the small, medium, and large L-shapes, and then returning to only the small L-shape, which may repeat.

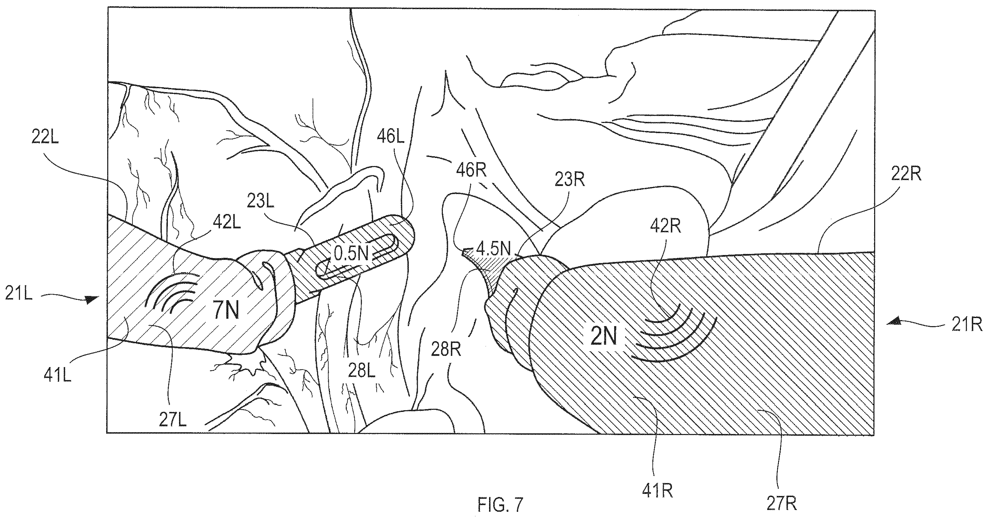

As shown in FIG. 7, each of the first direction representations 42R, 42L may be represented as a group of four arc-shapes indicating the direction of the corresponding force. The number of the arc-shapes may be one, two, three, five or more.

In the group, the arc-shapes with different sizes are aligned in order of the size. The direction indicated by the bisector of the central angle of each arc-shape represents the direction of the corresponding force. If there are two or more arc-shapes, the direction of the corresponding force of each arc-shape is also represented by a direction of arrangement from a small arc-shape to a large arc-shape.

A representation state of the group of the four arc-shapes may remain constant regardless of a passage of time, or may be changed over time in a manner similar to the group of the L-shapes. Specifically, as time passes, the representation may change from displaying only the small arc-shape to displaying sequentially adding larger arc-shapes thereto, and then return to only the small arc-shape, which may repeat.

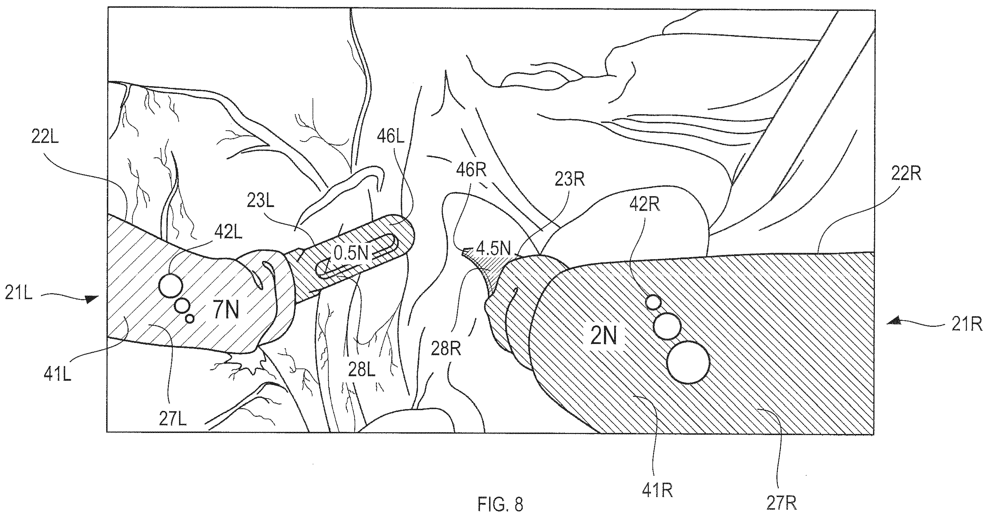

As shown in FIG. 8, each of the first direction representations 42R, 42L may be represented as a group of three circular-shapes indicating the direction of the corresponding force. The number of the circular-shapes may be two, four or more. In the group, the circular-shapes with different sizes are aligned in order of the size. The direction of the force is also represented by a direction of arrangement from a small circular-shape to a large circular-shape.

A representation state of the group of the three circular-shapes may remain constant regardless of a passage of time, or may be changed over time in a manner similar to the group of the L-shapes. Specifically, as time passes, the representation may change from displaying only the small circular-shape to displaying sequentially adding larger circular-shapes thereto, and then return to only the small circular-shape, which may repeat.

When the first surgical instrument area 27R and the second surgical instrument area 28R are detected and the first force sense representation and the second force sense representation are generated, the superimposing section 55 executes an arithmetic process to generate display information (S105: superimposing step), as shown in FIG. 9. In the present embodiment, as shown in FIG. 4, the superimposing section 55 executes an arithmetic process to generate display information for display of the first force amount representation 41R and the first direction representation 42R superimposed on the first surgical instrument area 27R. Further, the superimposing section 55 executes an arithmetic process to generate display information for display of the second force amount representation 46R superimposed on the second surgical instrument area 28R.

Preferably, the first force amount representation 41R and the first direction representation 42R are displayed so as to fit within the first surgical instrument area 27R. Preferably, the second force amount representation 46R is displayed so as to fit within the second surgical instrument area 28R.

The first force amount representation 41R and the first direction representation 42R may be displayed to partly extend beyond the first surgical instrument area 27R. The second force amount representation 46R may be displayed to partly extend beyond the second surgical instrument area 28R.

As shown in FIG. 9, when the display information is generated, the outputter 56 executes a process to output the display information to the endoscope monitor 12 (S106). The endoscope monitor 12 displays an image as shown in FIG. 4, based on the display information that is input.

As shown in FIG. 9, when the display information is output, the force sense display device 50 executes a process to determine whether a termination of displaying the force sense is input (S107). If the termination of displaying the force sense is not input (in a case of "NO"), the force sense display device 50 returns to S101 and repeats the aforementioned processing steps. If the termination of displaying the force sense is input (in a case of "YES"), the force sense display device 50 terminates the processing steps to display the force sense.

According to the aforementioned force sense display device 50, the first force amount representation 41R and the first direction representation 42R are displayed in a manner superimposed on the first surgical instrument area 27R, and the second force amount representation 46R is displayed in a manner superimposed on the second surgical instrument area 28R. The first force amount representation 41L and the first direction representation 42L are displayed in a manner superimposed on the first surgical instrument area 27L, and the second force amount representation 46L is displayed in a manner superimposed on the second surgical instrument area 28L.

Accordingly, it is easier to know the magnitude and direction of the corresponding force applied by each of the instruments 21R, 21L to the affected part. In other words, it is easier to know damage caused by each of the instruments 21R, 21L to the tissue in the affected part. As a result, it is easier to reduce the damage to the tissue.

Further, the first force amount representation 41R and the first direction representation 42R, and the second force amount representation 46R are respectively displayed in a manner superimposed on the first surgical instrument area 27R and the second surgical instrument area 28R. The first force amount representation 41L and the first direction representation 42L, and the second force amount representation 46L are respectively displayed in a manner superimposed on the first surgical instrument area 27L and the second surgical instrument area 28L. Accordingly, the affected part displayed on the endoscope monitor 12 is less likely to be hidden by the first force sense representation and the second force sense representation. The instruments 21R, 21L and the affected part are less likely to accidentally touch each other, and the damage to the tissue due to the touch is less likely to be reduced.

The first force amount representation 41R and the first direction representation 42R are displayed in a manner superimposed on the first surgical instrument area 27R, and the first force amount representation 41L and the first direction representation 42L are displayed in a manner superimposed on the first surgical instrument area 27L. Accordingly, it is easier to know the magnitude of and the direction of the corresponding force applied by each of the shafts 22R, 22L to the affected part.

The second force amount representation 46R is displayed in a manner superimposed on the second surgical instrument area 28R, and the second force amount representation 46L is displayed in a manner superimposed on the second surgical instrument area 28L. Accordingly, it is easier to know the magnitude of the corresponding force applied by each of the end effectors 23R, 23L to the affected part.

Representing, using a color type, the first force amount representation 41R and the second force amount representation 46R, each of which is the magnitude of the corresponding force, makes it easier to intuitively know the magnitude of the force.

Representing, using numerical values, the first force amount representation 41R and the second force amount representation 46R, each of which is the magnitude of the corresponding force, enables the magnitude of the force to be accurately represented.

Representing, using the size of the numerical values, the first force amount representation 41R and the second force amount representation 46R, each of which is the magnitude of the corresponding force, enables the magnitude of the force to be accurately represented and more easily known intuitively, compared to a case in which the magnitude of the force is represented using numerical values alone.

In the aforementioned embodiment, as an example, the case is described in which the force sense display device 50 causes the first force sense representations containing the first force amount representations 41R, 41L and the first direction representations 42R, 42L and the second force sense representations containing the second force amount representations 46R, 46L to be displayed on the endoscope monitor 12 in a superimposed manner. However, only either the first force sense representations or the second force sense representations may be displayed on the endoscope monitor 12 in a superimposed manner.

The second force sense representation may contain a second direction representation representing a direction of the second force, in addition to the second force amount representations 46R, 46L. In this case, the second force sense representation containing each of the second force amount representations 46R, 46L and the second direction representation is displayed on the endoscope monitor 12 in a superimposed manner.

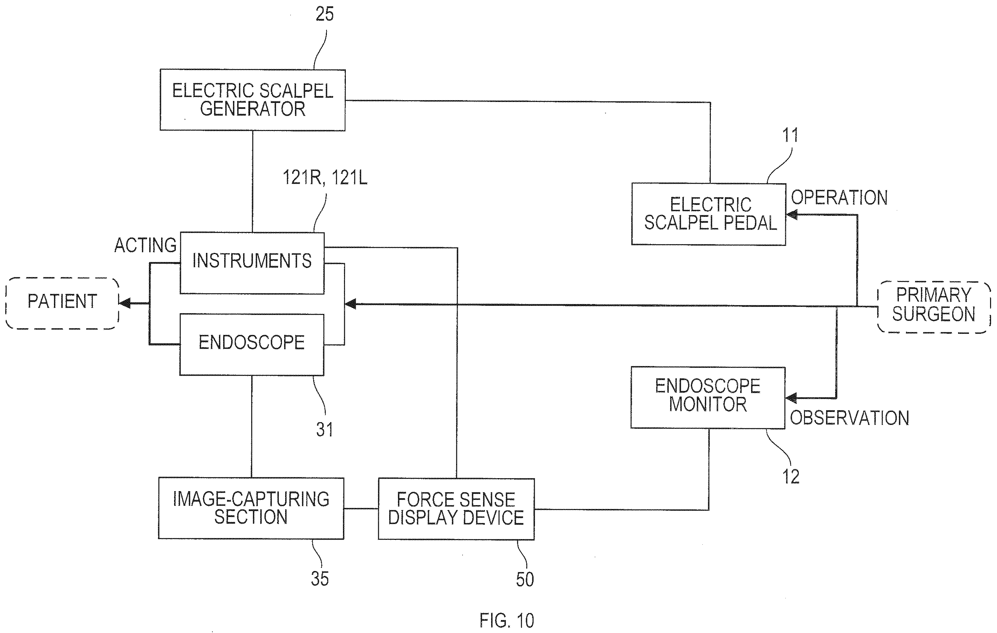

In the aforementioned embodiment, the example is described in which the force sense display device 50 is applied to surgery using the medical robot 1. However, the force sense display device 50 may be applied to laparoscopic surgery as shown in FIG. 10.

In a case of application to the laparoscopic surgery, two instruments 121R, 121L as surgical instruments are configured to be operated directly by the primary surgeon, unlike the instruments 21R, 21L operated by the slave 20.

The instrument 121R is provided with at least the shaft 22R, the end effector 23R, the shaft sensor 61R, and the end effector sensor 62R. The instrument 121L is provided with at least the shaft 22L, the end effector 23L, the shaft sensor 61L, and the end effector sensor 62L.

### [Modified Example of First Embodiment]

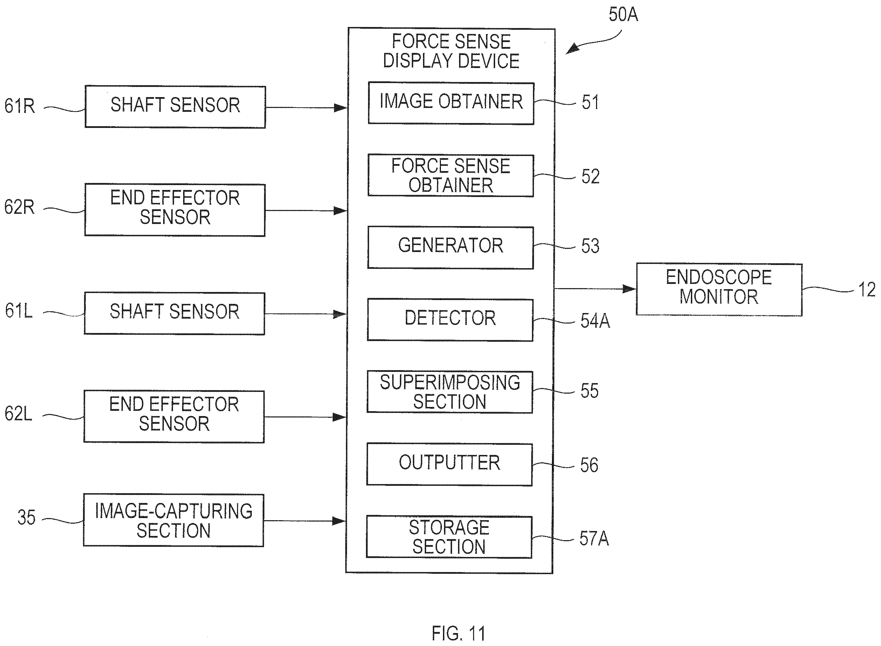

Next, a force sense display device 50A according to a modified example in the first embodiment of the present disclosure will be described with reference to FIG. 11.

The force sense display device 50A in the modified example has a basic configuration different from the first embodiment in the process to detect the surgical instrument area. Accordingly, in this embodiment, the process to detect the surgical instrument area is described with reference to FIG. 11, and other description will be omitted.

As shown in FIG. 11, the force sense display device 50A in the modified example is an information processing device such as a processor including a CPU (Central Processing Unit), a ROM, a RAM, an input/output interface, etc., a microcomputer, a personal computer, or a server.

A program stored in the storage device such as the ROM causes the CPU, the ROM, the RAM, and the input/output interface to cooperate with each other and function as at least the image obtainer 51, the force sense obtainer 52, the generator 53, a detector 54A, the superimposing section 55, the outputter 56, and a storage section 57A.

The storage section 57A stores at least one of a color tone or a pattern of each of the instruments 121R, 121L. The present embodiment describes an example configuration in which the storage section 57A stores the colors applied to the shaft 22R, the end effector 23R, the shaft 22L, and the end effector 23L. The storage section 57A may store a combination of the corresponding color tone and pattern of each of the instruments 121R, 121L, or may store patterns thereof.

Preferably, the corresponding color tone of each of the instruments 121R, 121L is complementary to a color of the affected part. For example, a complementary color to a color of blood is preferably, for example, green. Examples of the color of blood include a color of arterial blood and a color of venous blood.

Preferably, the pattern of each of the instruments 121R, 121L is a geometric pattern that is easily contrasted with the affected part. For example, preferably, a straight-line geometric pattern, or a pattern in which similar shapes repeatedly appear.

The detector 54A is a calculator that detects the surgical instrument area based on the obtained captured-image information and the information on the stored the corresponding color tone of each of the instruments 121R, 121L. As the image analysis process to detect the surgical instrument area based on the information on the color tone, a commonly-used process method for detecting a specific area based on information on a color tone may be used.

When the color tone of the instrument 121R and the color tone of the instrument 121L differ from each other, it is easier to detect the surgical instrument area of the instrument 121R and the surgical instrument area of the instrument 121L as areas different from each other.

Further, when the color tone of the shaft 22R and the color tone of the end effector 23R differ from each other, it is easier to detect the first surgical instrument area 27R and the second surgical instrument area 28R as areas different from each other. Similarly, when the color tone of the shaft 22L and the color tone of the end effector 23L differ from each other, it is easier to detect the first surgical instrument area 27L and the second surgical instrument area 28L as areas different from each other.

Next, a method for displaying a force sense in the force sense display device 50A having the aforementioned configuration is similar to the method for displaying the force sense in the first embodiment, except for having a different image analysis process to detect the surgical instrument area, and therefore, the description will be omitted.

According to the aforementioned force sense display device 50A, by detecting the first surgical instrument area 27R, the second surgical instrument area 28R, the first surgical instrument area 27L, and the second surgical instrument area 28L based on the corresponding color tone of each of the instruments 121R, 121L, it is easier to detect the first surgical instrument area 27R, the second surgical instrument area 28R, the first surgical instrument area 27L, and the second surgical instrument area 28L. Further, it is easier to more accurately detect each of the first surgical instrument area 27R, the second surgical instrument area 28R, the first surgical instrument area 27L, and the second surgical instrument area 28L.

## Claims

1. A force sense display device (50, 50A), comprising:
an image obtainer (51) configured to obtain captured-image information that is output from an image-capturing section (35) configured to capture an image of an affected part;
a force sense obtainer (52) configured to obtain force sense information comprising information on a magnitude of and a direction of a force acting on at least one surgical instrument (21R, 21L, 121R, 121L) for treatment of the affected part;
a generator (53) configured to generate a force sense representation based on the force sense information, the force sense representation being a mode of representation to be displayed on a display section (12) and comprising a force amount representation (41R, 41L, 46R, 46L) that represents the magnitude of the force and a direction representation (42R, 42L) that represents the direction of the force;
a detector (54, 54A) configured to detect a surgical instrument area (27R, 27L, 28R, 28L) where the surgical instrument (21R, 21L, 121R, 121L) is displayed in an image displayed on the display section (12), based on the captured-image information; and
a superimposing section (55) configured to generate display information for display, on the display section (12), of the surgical instrument area (27R, 27L, 28R, 28L) with the force sense representation superimposed thereon,
wherein the force sense information comprises information on a magnitude of and a direction of a first force acting on a shaft (22R, 22L) of the surgical instrument (21R, 21L, 121R, 121L) extending in a rod shape,
wherein the generator (53) generates a first force sense representation comprising a first force amount representation (41R, 41L) that represents the magnitude of the first force and a first direction representation (42R, 42L) that represents the direction of the first force,
wherein the detector (54, 54A) detects, as a first surgical instrument area (27R, 27L), an area where the shaft (22R, 22L) is displayed in the image displayed on the display section (12), and
wherein the superimposing section (55) generates display information for display, on the display section (12), of the first surgical instrument area (27R, 27L) with the first force sense representation superimposed thereon,
wherein the force sense information comprises information on a magnitude of and a direction of a second force applied to an end effector (23R, 23L) that is provided on an end of the shaft (22R, 22L) and used for treatment of the affected part,
wherein the generator (53) generates a second force sense representation comprising a second force amount representation (46R, 46L) that represents the magnitude of the second force and a second direction representation that represents the direction of the second force,
wherein the detector (54, 54A) detects, as a second surgical instrument area (28R, 28L), an area where the end effector (23R, 23L) is displayed in the image displayed on the display section (12), and
wherein the superimposing section (55) generates display information for display, on the display section (12), of the second surgical instrument area (28R, 28L) with the second force sense representation superimposed thereon.

2. The force sense display device (50, 50A) according to claim 1, wherein the generator (53) generates the force sense representation comprising the force amount representation (41R, 41L, 46R, 46L) that represents the magnitude of the force using at least one of a color type, a color brightness/darkness, or a transparency.

3. The force sense display device (50, 50A) according to claim 1 or 2, wherein the generator (53) generates the force sense representation comprising the force amount representation (41R, 41L, 46R, 46L) that represents the magnitude of the force using a numerical value.

4. The force sense display device (50, 50A) according to claim 3, wherein the generator (53) generates the force sense representation comprising the force amount representation (41R, 41L, 46R 46L) that represents the magnitude of the force using a size of the numerical value to be displayed.

5. The force sense display device (50A) according to any one of claims 1 to 4, further comprising a storage section (57A) configured to store at least one of a color tone or a pattern of the surgical instrument (121R, 121L),
wherein the detector (54A) detects the surgical instrument area (27R, 27L, 28R, 28L) in the image displayed on the display section (12), based on the at least one of the color tone or the pattern of the surgical instrument (121R, 121L) stored in the storage section (57A).

6. A program for causing a computer to execute:
an image obtaining function of obtaining captured-image information that is output from an image-capturing section (35) configured to capture an image of an affected part;
a force sense obtaining function of obtaining force sense information comprising information on a magnitude of and a direction of a force acting on at least one surgical instrument (21R, 21L, 121R, 121L) for treatment of the affected part;
a generating function of generating a force sense representation based on the force sense information, the force sense representation being a mode of representation to be displayed on a display section (12) and comprising a force amount representation (41R, 41L, 46R, 46L) that represents the magnitude of the force and a direction representation (42R, 42L) that represents the direction of the force;
a detecting function of detecting a surgical instrument area (27R, 27L, 28R, 28L) where the surgical instrument (21R, 21L, 121R, 121L) is displayed in an image displayed on the display section, based on the captured-image information; and
a superimposing function of generating display information for display, on the display section (12), of the surgical instrument area (27R, 27L, 28R, 28L) with the force sense representation superimposed thereon,
wherein the force sense information comprises information on a magnitude of and a direction of a first force acting on a shaft (22R, 22L) of the surgical instrument (21R, 21L, 121R, 121L) extending in a rod shape,
wherein the generating function generates a first force sense representation comprising a first force amount representation (41R, 41L) that represents the magnitude of the first force and a first direction representation (42R, 42L) that represents the direction of the first force,
wherein the detecting function detects, as a first surgical instrument area (27R, 27L), an area where the shaft (22R, 22L) is displayed in the image displayed on the display section (12), and
wherein the superimposing function generates display information for display, on the display section (12), of the first surgical instrument area (27R, 27L) with the first force sense representation superimposed thereon,
wherein the force sense information comprises information on a magnitude of and a direction of a second force applied to an end effector (23R, 23L) that is provided on an end of the shaft (22R, 22L) and used for treatment of the affected part,
wherein the generating function generates a second force sense representation comprising a second force amount representation (46R, 46L) that represents the magnitude of the second force and a second direction representation that represents the direction of the second force,
wherein the detecting function detects, as a second surgical instrument area (28R, 28L), an area where the end effector (23R, 23L) is displayed in the image displayed on the display section (12), and
wherein the superimposing function generates display information for display, on the display section (12), of the second surgical instrument area (28R, 28L) with the second force sense representation superimposed thereon.

## Patentansprüche

1. Krafterfassungsanzeigevorrichtung (50, 50A), enthaltend:
einen Bilderhalter (51), der konfiguriert ist, aufgenommene Bildinformationen zu erhalten, die aus einem Bildaufnahmeabschnitt (35) ausgegeben werden, der konfiguriert ist, ein Bild eines betroffenen Teils aufzunehmen;
einen Krafterfassungserhalter (52), der konfiguriert ist, Krafterfassungsinformationen zu erhalten, die Informationen über eine Größe und eine Richtung einer auf mindestens ein chirurgisches Instrument wirkenden Kraft (21R, 21L, 121R, 121L) zur Behandlung des betroffenen Teils enthalten;
einen Erzeuger (53), der konfiguriert ist, eine Krafterfassungsdarstellung auf Basis der Krafterfassungsinformationen zu erzeugen, wobei die Krafterfassungsdarstellung eine Darstellungsform ist, die auf einem Anzeigeabschnitt (12) anzuzeigen ist und die eine Kraftmengendarstellung (41R, 41L, 46R, 46L), die die Größe der Kraft darstellt, und eine Richtungsdarstellung (42R, 42L) enthält, die die Richtung der Kraft darstellt;
einen Detektor (54, 54A), der konfiguriert ist, eine chirurgische Instrumentenfläche (27R, 27L, 28R, 28L) zu detektieren, in der das chirurgische Instrument (21R, 21L, 121R, 121L) in einem Bild angezeigt wird, das im Anzeigeabschnitt (12) angezeigt wird, basierend auf den aufgenommenen Bildinformationen; und
einen Überlagerungsabschnitt (55), der konfiguriert ist, Anzeigeinformationen zur Anzeige, auf dem Anzeigeabschnitt (12) der chirurgischen Instrumentenfläche (27R, 27L, 28R, 28L) mit darauf überlagerter Krafterzeugungsdarstellung zu erzeugen,
wobei die Krafterfassungsinformationen Informationen über eine Größe und eine Richtung einer ersten Kraft enthalten, die auf einen Schaft (22R, 22L) des chirurgischen Instruments (21R, 21L, 121R, 121L) wirkt, der sich in einer Stangenform erstreckt,
wobei der Erzeuger (53) eine erste Krafterfassungsdarstellung erzeugt, die eine erste Kraftmengendarstellung (41R, 41L), die die Größe der ersten Kraft darstellt, und eine erste Richtungsdarstellung (42R, 42L) enthält, die die Richtung der ersten Kraft darstellt,
wobei der Detektor (54, 54A) als eine erste chirurgische Instrumentenfläche (27R, 27L) eine Fläche detektiert, in der der Schaft (22R, 22L) im Bild angezeigt wird, das im Anzeigeabschnitt (12) angezeigt wird, und
wobei der Überlagerungsabschnitt (55) Anzeigeinformationen zur Anzeige, auf dem Anzeigeabschnitt (12) der ersten chirurgischen Instrumentenfläche (27R, 27L) mit der ersten Krafterfassungsdarstellung darauf überlagert erzeugt,
wobei die Krafterfassungsinformationen Informationen über eine Größe und eine Richtung einer zweiten Kraft enthalten, die auf einen Endeffektor (23R, 23L) angewendet wird, der an einem Ende des Schafts (22R, 22L) bereitgestellt ist und zur Behandlung des betroffenen Teils verwendet wird,
wobei der Erzeuger (53) eine zweite Krafterfassungsdarstellung erzeugt, die eine zweite Kraftmengendarstellung (46R, 46L), die die Größe der zweiten Kraft darstellt, und eine zweite Richtungsdarstellung enthält, die die Richtung der zweiten Kraft darstellt,
wobei der Detektor (54, 54A) als eine zweite chirurgische Instrumentenfläche (28R, 28L) eine Fläche detektiert, in der der Endeffektor (23R, 23L) im Bild angezeigt wird, das im Anzeigeabschnitt (12) angezeigt wird, und
wobei der Überlagerungsabschnitt (55) Anzeigeinformationen zur Anzeige, auf dem Anzeigeabschnitt (12) der zweiten chirurgischen Instrumentenfläche (28R, 28L) mit der zweiten Krafterfassungsdarstellung darauf überlagert, erzeugt.

2. Krafterfassungsanzeigevorrichtung (50, 50A) gemäß Anspruch 1, wobei der Erzeuger (53) die Krafterfassungsdarstellung erzeugt, die die Kraftmengendarstellung (41R, 41L, 46R, 46L) enthält, die die Größe der Kraft unter Verwendung mindestens einer Farbart, einer Farbhelligkeit/-dunkelheit oder einer Transparenz darstellt.

3. Krafterfassungsanzeigevorrichtung (50, 50A) gemäß Anspruch 1 oder 2, wobei der Erzeuger (53) die Krafterfassungsdarstellung erzeugt, die die Kraftmengendarstellung (41R, 41L, 46R, 46L) enthält, die die Größe der Kraft unter Verwendung eines numerischen Werts darstellt.

4. Krafterfassungsanzeigevorrichtung (50, 50A) gemäß Anspruch 3, wobei der Erzeuger (53) die Krafterfassungsdarstellung erzeugt, die die Kraftmengendarstellung (41R, 41L, 46R 46L) enthält, die die Größe der Kraft unter Verwendung einer Größenordnung des anzuzeigenden Zahlenwerts darstellt.

5. Krafterfassungsanzeigevorrichtung (50A) gemäß einem der Ansprüche 1 bis 4, weiterhin enthaltend einen Speicherabschnitt (57A), der konfiguriert ist, mindestens einen Farbton oder ein Muster des chirurgischen Instruments (121R, 121L) zu speichern,
wobei der Detektor (54A) die chirurgische Instrumentenfläche (27R, 27L, 28R, 28L) im Bild detektiert, das im Anzeigeabschnitt (12) angezeigt wird, basierend auf mindestens einem bzw. eines des Farbtons oder des Musters des chirurgischen Instruments (121R, 121L), das im Speicherabschnitt (57A) gespeichert ist.

6. Programm zum Veranlassen eines Computers, auszuführen:
eine Bilderhaltungsfunktion zum Erhalten von aufgenommenen Bildinformationen, die aus einem Bildaufnahmeabschnitt (35) ausgegeben werden, der konfiguriert ist, ein Bild eines betroffenen Teils aufzunehmen;
eine Krafterfassungserhaltungsfunktion zum Erhalten von Krafterfassungsinformationen, die Informationen über eine Größe und eine Richtung einer auf mindestens ein chirurgisches Instrument wirkenden Kraft (21R, 21L, 121R, 121L) zur Behandlung des betroffenen Teils enthalten;
eine Erzeugungsfunktion zum Erzeugen einer Krafterfassungsdarstellung basierend auf den Krafterfassungsinformationen, wobei die Krafterfassungsdarstellung eine Darstellungsform ist, die auf einem Anzeigeabschnitt (12) anzuzeigen ist und eine Kraftmengendarstellung (41R, 41L, 46R, 46L), die die Größe der Kraft darstellt, und eine Richtungsdarstellung (42R, 42L) enthält, die die Richtung der Kraft darstellt;
eine Detektionsfunktion zum Detektieren einer chirurgischen Instrumentenfläche (27R, 27L, 28R, 28L), in der das chirurgische Instrument (21R, 21L, 121R, 121L) in einem Bild angezeigt wird, das auf dem Anzeigeabschnitt angezeigt wird, basierend auf den aufgenommenen Bildinformationen; und
eine Überlagerungsfunktion zum Erzeugen von Anzeigeinformationen zur Anzeige auf dem Anzeigeabschnitt (12) der chirurgischen Instrumentenfläche (27R, 27L, 28R, 28L) mit der darauf überlagerten Krafterfassungsdarstellung,
wobei die Krafterfassungsinformationen Informationen über eine Größe und eine Richtung einer ersten Kraft enthalten, die auf einen Schaft (22R, 22L) des chirurgischen Instruments (21R, 21L, 121R, 121L) wirkt, der sich in einer Stangenform erstreckt,
wobei die Erzeugungsfunktion (53) eine erste Krafterfassungsdarstellung erzeugt, die eine erste Kraftmengendarstellung (41R, 41L), die die Größe der ersten Kraft darstellt, und eine erste Richtungsdarstellung (42R, 42L) enthält, die die Richtung der ersten Kraft darstellt,
wobei die Detektionsfunktion (54, 54A) als eine erste chirurgische Instrumentenfläche (27R, 27L) eine Fläche detektiert, in der der Schaft (22R, 22L) im Bild angezeigt wird, das im Anzeigeabschnitt (12) angezeigt wird, und
wobei die Überlagerungsfunktion (55) Anzeigeinformationen zur Anzeige, auf dem Anzeigeabschnitt (12) der ersten chirurgischen Instrumentenfläche (27R, 27L) mit der ersten Krafterfassungsdarstellung darauf überlagert erzeugt,
wobei die Krafterfassungsinformationen Informationen über eine Größe und eine Richtung einer zweiten Kraft enthalten, die auf einen Endeffektor (23R, 23L) angewendet wird, der an einem Ende des Schafts (22R, 22L) bereitgestellt ist und zur Behandlung des betroffenen Teils verwendet wird,
wobei die Erzeugungsfunktion (53) eine zweite Krafterfassungsdarstellung erzeugt, die eine zweite Kraftmengendarstellung (46R, 46L), die die Größe der zweiten Kraft darstellt, und eine zweite Richtungsdarstellung enthält, die die Richtung der zweiten Kraft darstellt,
wobei die Detektionsfunktion (54, 54A) als eine zweite chirurgische Instrumentenfläche (28R, 28L) eine Fläche detektiert, in der der Endeffektor (23R, 23L) im Bild angezeigt wird, das im Anzeigeabschnitt (12) angezeigt wird, und
wobei die Überlagerungsfunktion (55) Anzeigeinformationen zur Anzeige, auf dem Anzeigeabschnitt (12) der zweiten chirurgischen Instrumentenfläche (28R, 28L) mit der zweiten Krafterfassungsdarstellung darauf überlagert, erzeugt.

## Revendications

1. Un dispositif d'affichage de détection de force (50, 50A), comprenant :
un obteneur d'image (51) configuré pour obtenir des informations d'image capturée provenant d'une section capturant l'image (35) configurée pour capturer une image d'une pièce affectée ;
un obteneur de détection de force (52) configuré pour obtenir des informations de détection de force comprenant des informations sur une magnitude et une direction d'une force agissant sur au moins un instrument chirurgical (21R, 21L, 121R, 121L) pour le traitement de la pièce affectée ;
un générateur (53) configuré pour générer une représentation de détection de force basée sur l'information de détection de force, la représentation de détection de force étant un mode de représentation à afficher sur une section d'affichage (12) et comprenant une représentation de magnitude de force (41R, 41L, 46R, 46L) représentant la magnitude de la force et une représentation de direction (42R, 42L) représentant la direction de la force ;
un détecteur (54, 54A) configuré pour détecter une zone d'instrument chirurgical (27R, 27L, 28R, 28L) où l'instrument chirurgical (21R, 21L, 121R, 121L) est affiché dans une image affichée dans la section d'affichage (12), basée sur les informations d'image capturée ; et
une section de superposition (55) configurée pour générer des informations d'affichage à afficher, sur la section d'affichage (12), de la zone de l'instrument chirurgical (27R, 27L, 28R, 28L) avec la représentation de détection de force superposée dessus,
dans lequel l'information de détection de force comprend des informations sur une magnitude et une direction d'une première force agissant sur un arbre (22R, 22L) de l'instrument chirurgical (21R, 21L, 121R, 121L) s'étendant en forme de tige,
dans lequel le générateur (53) génère une première représentation de détection de force comprenant une première représentation de magnitude de force (41R, 41L) qui représente la magnitude de la première force et une première représentation de direction (42R, 42L) qui représente la direction de la première force,
dans lequel le détecteur (54, 54A) détecte, en tant que première zone d'instrument chirurgical (27R, 27L), une zone où l'arbre (22R, 22L) est affiché dans l'image affichée dans la section d'affichage (12), et
dans lequel la section de superposition (55) génère des informations d'affichage pour afficher, sur la section d'affichage (12), de la première zone d'instrument chirurgical (27R, 27L) avec la première représentation de détection de force superposée dessus,
dans lequel l'information de détection de force comprend des informations sur une magnitude et une direction d'une seconde force appliquée à un effecteur d'extrémité (23R, 23L) fourni à une extrémité de l'arbre (22R, 22L) et utilisé pour traiter la pièce affectée,
dans lequel le générateur (53) génère une seconde représentation de détection de force comprenant une seconde représentation de magnitude de force (46R, 46L) qui représente la magnitude de la seconde force et une seconde représentation de direction qui représente la direction de la seconde force,
dans lequel le détecteur (54, 54A) détecte, comme une seconde zone d'instrument chirurgical (28R, 28L), une zone où l'effecteur d'extrémité (23R, 23L) est affiché dans l'image affichée sur la section d'affichage (12), et
dans lequel la section de superposition (55) génère des informations d'affichage pour afficher, dans la section d'affichage (12), de la seconde zone de l'instrument chirurgical (28R, 28L), avec la seconde représentation de détection de force superposée dessus.

2. Le dispositif d'affichage de détection de force (50, 50A) selon la revendication 1, dans lequel le générateur (53) génère la représentation de détection de force comprenant la représentation de la magnitude de force (41R, 41L, 46R, 46L) qui représente la magnitude de la force en utilisant au moins un de type de couleur, de luminosité/obscurité de couleur ou de transparence.

3. Le dispositif d'affichage de détection de force (50, 50A) selon la revendication 1 ou 2, dans lequel le générateur (53) génère la représentation de détection de force comprenant la représentation de magnitude de force (41R, 41L, 46R, 46L) qui représente la magnitude de la force en utilisant une valeur numérique.

4. Le dispositif d'affichage de détection de force (50, 50A) selon la revendication 3, dans lequel le générateur (53) génère la représentation de détection de force comprenant la représentation de magnitude de force (41R, 41L, 46R, 46L) qui représente la magnitude de la force en utilisant une dimension de la valeur numérique à afficher.

5. Le dispositif d'affichage de détection de force (50A) selon l'une des revendications 1 à 4, comprenant en outre une section de stockage (57A) configurée pour stocker au moins un de ton de couleur ou d'un motif de l'instrument chirurgical (121R, 121L),
dans lequel le détecteur (54A) détecte la zone de l'instrument chirurgical (27R, 27L, 28R, 28L) sur l'image affichée dans la section d'affichage (12), en se basant sur le au moins un de ton de couleur ou du motif de l'instrument chirurgical (121R, 121L) stockée dans la section de stockage (57A).

6. Un programme pour faire exécuter un ordinateur :
une fonction d'obtention d'image pour obtenir des informations d'image capturée qui sont sorties d'une section de capture d'image (35) configurée pour capturer une image d'une pièce affectée ;
une fonction d'obtention de détection de force pour obtenir des informations de détection de force comprenant des informations sur une magnitude et une direction d'une force agissant sur au moins un instrument chirurgical (21R, 21L, 121R, 121L) pour le traitement de la pièce affectée ;
une fonction de génération pour générer une représentation de détection de force basée sur l'information de détection de force, la représentation de détection de force étant un mode de représentation à afficher sur une section d'affichage (12) et comprenant une représentation de magnitude de force (41R, 41L, 46R, 46L) qui représente la magnitude de la force et une représentation de direction (42R, 42L) qui représente la direction de la force ;
une fonction de détection pour détecter la zone d'un instrument chirurgical (27R, 27L, 28R, 28L) où l'instrument chirurgical (21R, 21L, 121R, 121L) est affiché dans une image affichée dans la section d'affichage, basée sur l'information de l'image capturée ; et
une fonction de superposition pour générer des informations d'affichage pour afficher, sur la section d'affichage (12), de la zone de l'instrument chirurgical (27R, 27L, 28R, 28L) avec la représentation de détection de force superposée dessus,
dans lequel les informations de détection de force comprennent des informations sur une magnitude et une direction d'une première force agissant sur un arbre (22R, 22L) de l'instrument chirurgical (21R, 21L, 121R, 121L) s'étendant en forme de tige,
dans lequel la fonction de génération génère une première représentation de détection de force comprenant une première représentation de magnitude de force (41R, 41L) qui représente la magnitude de la première force et une première représentation de la direction (42R, 42L) qui représente la direction de la première force,
dans lequel la fonction de détection (54, 54A) détecte, en tant que première zone d'instrument chirurgical (27R, 27L), une zone où l'arbre (22R, 22L) est affiché dans l'image affichée dans la section d'affichage (12), et
dans lequel la fonction de superposition (55) génère des informations d'affichage pour afficher, sur la section d'affichage (12), de la première zone d'instrument chirurgical (27R, 27L) avec la première représentation de détection de force superposée dessus,
dans lequel les informations de détection de force comprennent des informations sur une magnitude et une direction d'une seconde force appliquée à un effecteur d'extrémité (23R, 23L) fourni à une extrémité de l'arbre (22R, 22L) et utilisé pour traiter la pièce affectée,
dans lequel la fonction de génération (53) génère une seconde représentation de détection de force comprenant une seconde représentation de magnitude de la force (46R, 46L) qui représente la magnitude de la seconde force et une seconde représentation de direction qui représente la direction de la seconde force,
dans lequel la fonction de détection (54, 54A) détecte, comme une seconde zone d'instrument chirurgical (28R, 28L), une zone où l'effecteur d'extrémité (23R, 23L) est affiché dans l'image affichée sur la section d'affichage (12), et
dans lequel la fonction de superposition (55) génère des informations d'affichage pour afficher, dans la section d'affichage (12), de la seconde zone de l'instrument chirurgical (28R, 28L), avec la seconde représentation de détection de force superposée dessus.
